Europäisches Patentamt

European Patent Office

Office européen des brevets

(11) Veröffentlichungsnummer: **0 270 908**
**A2**

## (12) EUROPÄISCHE PATENTANMELDUNG

(21) Anmeldenummer: **87117285.4**

(22) Anmeldetag: **24.11.87**

(51) Int. Cl.4: **C12N 5/02 , C12M 3/00 , C12P 21/00**

(30) Priorität: **06.12.86 DE 3641826**

(43) Veröffentlichungstag der Anmeldung:
**15.06.88 Patentblatt 88/24**

(84) Benannte Vertragsstaaten:
**AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Bödeker, Berthold, Dr.**
**Pahlkestrasse 17**
**D-5600 Wuppertal 1(DE)**
Erfinder: **Hewlett, Guy, Dr.**
**Krutscheider Weg 96**
**D-5600 Wuppertal 11(DE)**
Erfinder: **Schlumberger, Horst Dieter, Prof. Dr.**
**Schwartnerstrasse 5**
**D-5600 Wuppertal 2(DE)**
Erfinder: **Büntemeyer, Heino**
**Im Schabenkamp 26**
**D-3300 Braunschweig(DE)**
Erfinder: **Lehmann, Jürgen, Dr.**
**Boekenhainstrasse 20**
**D-3300 Braunschweig(DE)**

(54) **Verfahren zur Kultivierung von Säugerzellen in einer Suspensionskultur, Vorrichtung zur Durchführung des Verfahrens und Verwendung bei der Herstellung von Proteinen.**

(57) Die vorliegende Erfindung betrifft ein Verfahren zur Kultivierung von Säugerzellen in Suspensionskultur wobei der Mediumaustausch durch Ultrafiltration erfolgt. Das Kulturmedium wird zuerst über eie mikroporöse Membran aus dem Reaktor abgezogen um ein zellfreies Medium zu erhalten. Dieses Medium wird einer Ultrafiltrationsmembran zugeführt und ultrafiltriert. Das die niedermolekularen Bestandteile und Stoffwechselprodukte enthaltende Filtrat wird verworfen und das Konzentrat dem Reaktor wieder zugeführt. Aus einem Mediumreservoir wird dem Reaktor entsprechend der Menge an Filtrat frisches Medium zugeführt.

EP 0 270 908 A2

BAYER AKTIENGESELLSCHAFT        5090 Leverkusen, Bayerwerk

Konzernverwaltung RP

Patentabteilung        Bu/Ke-c

Verfahren zur Kultivierung von Säugerzellen in einer
Suspensionskultur, Vorrichtung zur Durchführung des
Verfahrens und Verwendung bei der Herstellung von
Proteinen
_____

Die vorliegende Erfindung betrifft ein Verfahren zur
Kultivierung von Säugerzellen in Suspensionskultur, wobei
der Mediumaustausch durch Ultrafiltration erfolgt. Das
Kulturmedium wird zuerst über eie mikroporöse Membran
aus dem Reaktor abgezogen um ein zellfreies Medium zu
erhalten. Dieses Medium wird einer Ultrafiltrationsmembran zugeführt und ultrafiltriert. Das die niedermolekularen Bestandteile und Stoffwechselprodukte enthaltende Filtrat wird verworfen und das Konzentrat dem
Reaktor wieder zugeführt. Aus einem Mediumreservoir wird
dem Reaktor entsprechend der Menge an Filtrat frisches
Medium zugeführt.

Bei der Kultivierung tierischer Zellen in Bioreaktoren
ergeben sich mehrere Probleme:

0 270 908

- 2 -

1) Die Kultur muß mit Gasen ($O_2$, $CO_2$) und Nährstoffen (flüssigem Medium mit Glukose, Aminosäuren, Vitaminen, Proteinen usw.) gut versorgt werden.

2) Abfallstoffe (Lactat, freigesetzte Aminosäuren, Ammoniak, im Überschuß gebildetes $CO_2$ usw.) müssen entsorgt werden.

Dies macht in der Regel bei hohen Zelldichten einen hohen Einsatz von Flüssigmedium und Sauerstoff erforderlich. Für die Lösung dieser Anforderungen gibt es inzwischen mehrere Wege. Zur Begasung werden Schlauchbegasung [Silikonschlauch: DE 2 940 446, blasenfreie Begasung mit porösen Polypropylenhohlfasern: DE 3 430 924 A1], Blasenbegasung oder Oberflächenbegasung eingesetzt.

Zum Mediumaustausch mit Zellrückhaltung werden Spinfilter (A.L. von Wezel (1984), Developments biol. Standard. 55, Seite 3-9), Membranen oder externe Kreislaufsysteme mit Filtereinheiten (W.R. Tolbert, Y . Feder (1983), Annual Reports on Fermentation Processes, Vol. 6, Seite 35-74) eingesetzt.

Aufgrund der geringen Produktivität von tierischen Zellkulturen ergeben sich nur geringe Produktkonzentrationen, da bei kontinuierlichem Betrieb hohe Mediumdurchsätze notwendig sind. Die Produkte müssen in weiteren Aufarbeitungsschritten aufkonzentriert werden. Dafür werden bekannte Verfahren (Ultrafiltration, Ionenaustauscher, Chromatographie, $(NH_4)_2SO_4$-Fällungen usw.) eingesetzt.

Le A 24 836

Die Trennung von Produktion und Produktkonzentrierung bedingt im Falle einer serumfreien Kultivierung eine ständige Erneuerung der Schutzproteine im zulaufenden Medium. Es bestand deshalb die Aufgabe, ein Verfahren und eine Vorrichtung zu entwickeln, bei der Kultivierung der Zellen, Produktion und Produktkonzentrierung kontinuierlich vorgenommen werden kann.

Gelöst wird diese Aufgabe dadurch, daß der Medienaustausch durch Ultrafiltration erfolgt. Durch die Ultrafiltration des Mediums werden die hochmolekularen Produkte im Kulturmedium zurückgehalten und die niedermolekularen Stoffwechselprodukte über das Filtrat entsorgt.

Die Ultrafiltration kann sowohl absatzweise als auch kontinuierlich erfolgen. Um das Volumen vom Kulturmedium konstant zu halten, wird aus einem Vorratsgefäß soviel frisches Medium der Kultur hinzugefügt, wie als Filtrat abgezogen wurde. Um eine Verstopfung der Ultrafiltrationsmembran zu vermeiden wird das Kulturmedium über eine mikroporöse hydrophilisierte Membran, die Zellen zurückhält, aus dem Reaktor entnommen. Geeignet sind z.B poröse Membranen aus Polypropylen (DE-A-3 107 874) die durch benetzen mit einem organischen Lösungsmittel und anschließend Wasserbehandlung hydrophilisiert werden. Das durch die Ultrafiltration gewonnene Konzentrat kann dem Reaktor wieder zugeführt oder zur Ernte des Produkts entnommen werden. Die Begasung des Kulturmediums mit Sauerstoff oder Luft erfolgt vorteilhafterweise nach dem in der DE-A 1 34 30 924 beschriebenen Verfahren. Als besonders geeignet für dieses Verfahren der Begasung haben sich Membranhohlfäden erwiesen die hydrophob und porös sind wie zum Beispiel Hohlfäden aus Polypropylen.

Unter "Suspensionskultur" im Rahmen der vorliegenden Erfindung werden Kulturen sowohl von Zellen verstanden die frei im Medium als Suspension wachsen, als auch Kulturen von adhärenten auf Mikrocarrien oder anderen Partikeln wachsenden Zellen.

Das erfindungsgemäße Verfahren besitzt die folgenden Vorteile:

1) Es ist möglich, sehr hohe Zelldichten $>1 \cdot 10^7$ Zellen/ml über lange Zeit kontinuierlich zur erreichen.

2) Durch die Akkumulation des gewünschten Produkts im Reaktorsystem kommt man zu sehr hohen Konzentrationen, die eine Endreinigung der Kultursuspension erleichtert.

3) Die hohe Produktkonzentration im Bioreaktor erlaubt den Einsatz proteinfreier Nährmedien.

4) Durch den absatzweisen oder kontinuierlichen Produktabzug aus dem Produktionsreaktor kann die Zellkultur auch bei hohen Zelldichten in einem guten Wachstumszustand gehalten werden, wodurch hohe spezifische Produktbildungsraten erreicht werden.

Le A 24 836

5) Die Vorteile homogener Zellkultivierung unter kontrollierten Kulturbedingungen (Sauerstoff, pH), die gleichmäßige Versorgung aller Zellen mit Medium und Entsorgung von Stoffwechselprodukten garantiert eine hohe Lebendzellzahl und nur einen geringen Anteil von toten Zellen im System.

6) Über die Wahl der Ausschlußgrenze der Ultrafiltrationseinheit kann die Qualität des zurückgeführten Anteils bestimmt werden. Die Ausschlußgrenze kann von < 1000 Dalton bis > 150 000 Dalton variabel sein. Sie muß so gewählt werden, daß die Membran für Substrate und inhibitorische Stoffwechselprodukte permeabel, für das Produkt jedoch impermeabel ist. Bevorzugt eingesetzt werden Membranen mit Ausschlußgrenzen von < 100 000 wie z.B. 5000, 10 000, 20 000 oder 50 000.

7) Durch das kontinuierliche Verfahren werden die notwendigen Reaktionsvolumen reduziert.

8) Es ist möglich, nicht nur Suspensionszellen, sondern auch adhärente Zellen, z.B. in Form der Microcarrierkultur, einzusetzen, da durch den zellfreien Mediumabzug verhindert wird, daß die Dialysemembran von adhärenten Zellen besiedelt wird, wodurch ihre Funktion zur Ultrafiltration beeinträchtigt wird.

Le A 24 836

- 6 -

### Beispiel

Die erwähnte Kombination von Mikrofiltration (im Reaktor) und Ultrafiltration (extern) wurde im 1,2 l-Maßstab getestet. Als Zellinie wurden humane EBV-transformierte B-Lymphozyten (E₄) in serumfreier Kultur eingesetzt. Diese Zelle produziert monoklonale Antikörper.

Das Medium bestand aus einer 1:1 Mischung der Basalmedien DMEM und F12 unter Zusatz der Proteine BSA (1 g/l), Transferrin (10 mg/l) und Insulin (10 mg/l) und einigen niedermolekularen Suppelementen.

In einen 1,2 l-Reaktor werden nacheinander 2 m der hydrophilisierten Polypropylenhohlfasermembran für den Mediumsaustausch und 2 m der hydrophoben Membran für die Begasung (DE 3 430 924 A1) eingebaut und im betriebsfertigen Reaktor sterilisiert. Die Begasung wurde über eine $pO_2$-Regeleinheit und ein 4-Kanal-Mass-Flow-Meter auf einen $pO_2$-Sollwert von 40 % Luftsättigung gehalten. Der pH-Wert konnte über eine $CO_2$-Einstellung im Begasungssystem geregelt werden.

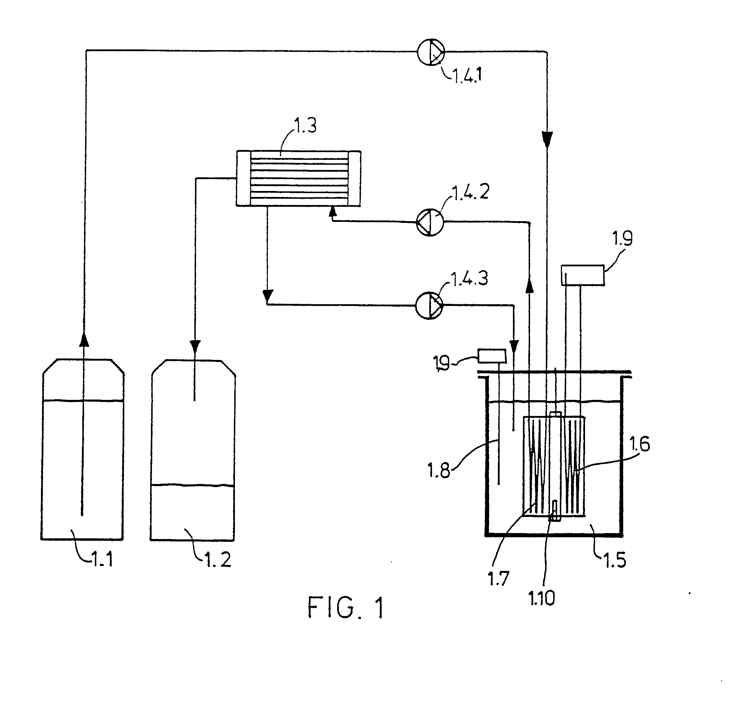

Das Nährmedium (BSA- und Transferrin frei) wurde absatzweise über eine Pumpe (1.4.1), siehe Fig. 1, durch die hydrophilisierte Membran in den Reaktor gepumpt und das verbrauchte Medium über eine zweite Pumpe (1.4.2) durch dieselbe Mebran entfernt. Während Pumpe 1.4.1 lief, stand Pumpe 1.4.2 und verschloß so den Ausgang der Hohlfasermembran. Beim Entfernen des Mediums durch Pumpe 1.4.2 stand Pumpe 1.4.1 und verschloß den Eingang der Membran. Dieser

Le A 24 836

Betrieb konnte über eine Gewichtsregelung gesteuert werden und garantierte einen störungsfreien Ablauf durch ständige Rückspülung der Membran mit frischem Medium.

Der Ausgang der Hohlfasermembran wurde über die Pumpe P2 an die Dialysatseite (Filtratseite) eines Dialysemoduls angeschlossen. Über die Pumpe 1.4.3 wird das Konzentrat trat aus der Dialysatseite entnommen und in den Reaktor zurückgeführt. Das Differenzvolumen, das sich aus den Pumpraten der Pumpen 1.4.2 und 1.4.3 ergab, wurde über die Ultrafiltrationsmembran gedrückt und im Ablaufbehälter gesammelt. Es betrug etwa 95 % des Gesamtstromes.

In den Zeichnungen 1 und 2 sind die Ergebnisse der Fermentation dargestellt.

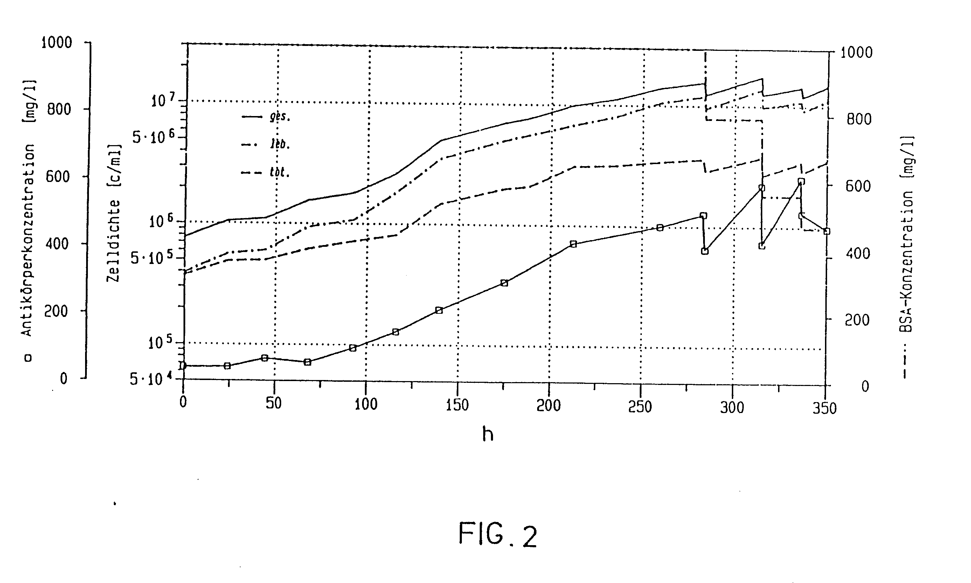

In Fig. 2 sind die Zelldichten und Proteinkonzentrationen (BSA und AK) aufgetragen. Der Fermenter wurde mit einer Gesamtzelldichte von $7,5 \cdot 10^5$ Z/ml und einem Anteil an toten Zellen von 48 % angeimpft. Der kontinuierliche Betrieb wurde kurz nach dem Animpfen gestartet. Innerhalb der ersten 80 Stunden wächst die Kultur auf eine Lebendzelldichte von etwa $1 \cdot 10^6$ Z/ml heran, wobei der Anteil der toten Zellen auf 30 % absinkt und die Antikörperkonzentration relativ konstant bei 50 bis 100 mg/l bleibt. Während der nächsten 60 Stunden zeigt die Kultur die kürzesten Verdoppelungszeiten. Der Anteil der toten Zellen sinkt auf 20 bis 25 % ab und bleibt von da an innerhalb dieser Grenzen. Die Antikörperkonzentration steigt jetzt linear an (Akkumulationseffekt der UF-Membran). Bei einer Lebendzelldichte von $3,5 \cdot 10^6$ Z/ml

Le A 24 836

- 8 -

knickt die Wachstumskurve auf eine neue, längere Verdoppelungszeit ab, bleibt aber danach bis zu einer Lebendzelldichte von $1,2 \cdot 10^7$ Z/ml bei der Fermentationszeit von 260 Stunden relativ konstant. Dabei steigt die Antikörperkonzentration auf 520 mg/l an.

Danach wurde eine Zell- und Produkternte von 21 % des Reaktorvolumens durchgeführt (Antikörperernte: 125 mg) und mit proteinfreiem Medium aufgefüllt. Nach 30 Stunden war die Lebendzelldichte auf $1,4 \cdot 10^7$ Z/ml und die Antikörperkonzentration auf 590 mg/l gestiegen. Es wurde erneut eine Zell- und Produkternte vorgenommen, diesmal von 29 %, und wieder mit proteinfreiem Medium aufgefüllt. Dabei wurden etwa 200 mg Antikörper geerntet. Nach weiteren 23 Stunden war die Lebendzelldichte wieder auf $1,2 \cdot 10^7$ Z/ml und die Antikörperkonzentration auf 610 mg/l angestiegen. Während der letzten 150 Stunden war der Mediendurchsatz auf etwa 0,9- bis 1,3-faches Reaktorvolumen pro Tag eingestellt.

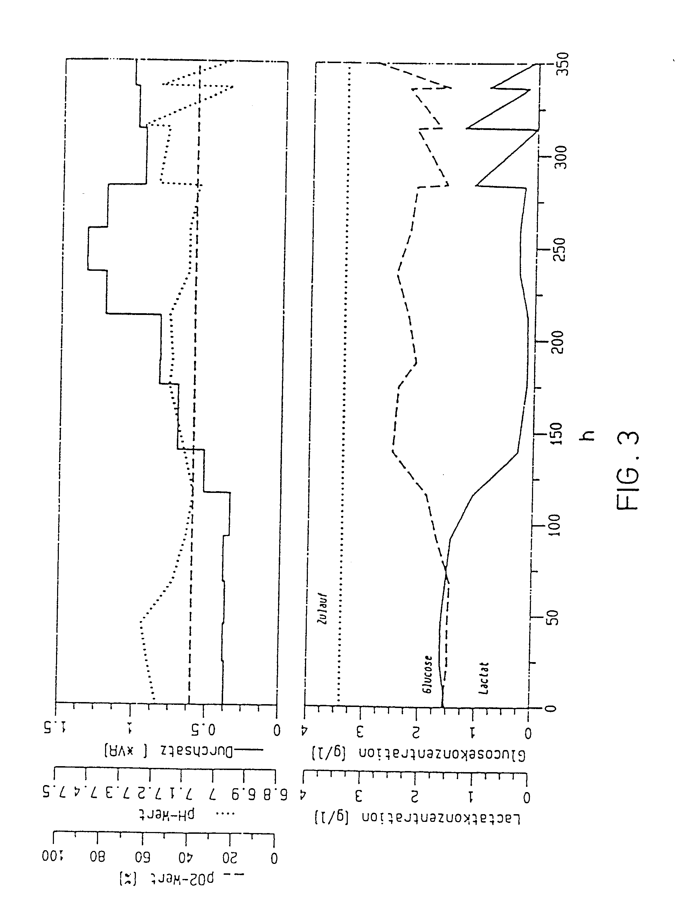

In Fig. 3 sind die weiteren Fermentationsparameter dargestellt (Mediendurchsatz, pH, $pO_2$, Glukose, Laktat).

Durch den Einsatz einer UF-Membran mit einer Ausschlußgrenze von 10.000 Dalton war nur eine einmalige Zugabe von BSA (67.000 Dalton) und Transferrin (87.000 Dalton) in den Reaktor notwendig. Eine Zugabe von Insulin in das Nährmedium wurde nur bis zu einer Lebendzelldichte von $1 \cdot 10^6$ Zellen/ml aufrechterhalten und danach vollständig abgesetzt. Die Zugabe der niedermolekularen Supplemente wurde während der gesamten Fermentation aufrechterhalten, aber auch bei hoher Lebendzelldichte nicht erhöht.

<u>Le A 24 836</u>

- 9 -

In Tabelle 1 sind die Proteinkonzentrationen im Reaktor und der prozentuale Anteil der Antikörper am Gesamtprotein wiedergegeben.

Tabelle 1

| T [h] | BSA [mg/l] | IgM [mg/l] | % IgM |
|-------|-----------|------------|-------|
| 116 | 1.000 | 150 | 13 |
| 283 | 1.000 | 505 | 34 |
| 313 | 790 | 589 | 43 |
| 336 | 560 | 610 | 52 |

Beschreibung der Zeichnungen

Fig. 1:   Diese Zeichnung zeigt schematisch den Aufbau der erfindungsgemäßen Vorrichtung

1.1   Vorratsgefäß für frisches Medium

1.2   Gefäß für Filtrat

1.3   Ultrafiltrationsmodul

1.4.1-1.4.3 Pumpen

1.5 Reaktor

1.6 Hydrophobe Begasungsmembran mit Gasversorgung

1.7 Hydrophilisierte Hohlfasermembran für den Mediumaustausch

1.8 pH-Elektrode

1.9 Kontrolleinheiten für pH, $pO_2$, $pCO_2$ etc.

1.10 Halterung für die Begasungs- und Mediumaustauschmembran

0 270 908

- 10 -

Fig. 2: Abhängigkeit der Antikörperkonzentrationen, BSA·Konzentration und der Zelldichte von der Kultivierungszeit.

Fig. 3: Abhängigkeit der Konzentration von Nährstoffen und Stoffwechselprodukten von der Kultivierungszeit.

Le A 24 836

- 11 -

Patentansprüche

1. Verfahren zur Kultivierung von Säugerzellen in einer Suspensionskultur dadurch gekennzeichnet, daß der Medienaustausch durch Ultrafiltration erfolgt.

2. Verfahren nach Anspruch 1, wobei das der Ultrafiltration zugeführte Medium ein zellfreies Filtrat aus dem Reaktor ist.

3. Verfahren gemäß den Ansprüchen 1 und 2, wobei Sauerstoffkonzentration und pH-Wert kontrolliert und reguliert werden.

4. Verfahren gemäß den Ansprüchen 1 bis 3, wobei die Ultrafiltrationsmembran undurchlässig ist für Moleküle mit einem Molekulargewicht von mehr als 100.000 Dalton.

5. Verfahren gemäß den Ansprüchen 1 bis 4, wobei die Ultrafiltrationsmembran undurchlässig ist für Moleküle mit einem Molekulargewicht von mehr als 50.000 Dalton.

6. Verfahren gemäß den Ansprüchen 1 bis 5, wobei die Ultrafiltrationsmembran undurchlässig ist für Moleküle mit einem Molekulargewicht von mehr als 10.000 Dalton.

7. Verfahren gemäß den Ansprüchen 1 bis 6, wobei der Medienaustausch durch die Ultrafiltration absatzweise oder kontinuierlich erfolgt.

Le A 24 836

- 12 -

8. Vorrichtung zur Durchführung des Verfahrens nach den Ansprüchen 1 bis 7, bestehend aus einem Reaktor für die in Suspension kultivierten Zellen, den für die Steuerung der Kultivierung notwendigen Kontrollsystemen und einem Filter zum abziehen von zellfreiem Medium aus dem Reaktor, wobei das zellfreie Medium in den Reaktor zurückgeführt wird, dadurch gekennzeichnet, daß in den Kreislauf für das zellfreie Medium eine Ultrafiltrationsmembran zum Mediumaustausch inkorporiert ist.

9. Vorrichtung nach Anspruch 8, zusätzlich enthaltend ein Mediumreservoir aus dem das durch die Ultrafiltration abgezogene Medium durch frisches Medium ersetzt wird.

10. Verwendung der Vorrichtung gemäß den Ansprüchen 8 und 9 bei der Herstellung von rekombinanten und natürlichen Proteinen, Enzymen und Antikörpern.

Le A 24 836

FIG. 1

FIG. 2

FIG. 3

0 270 908